# FASCICULE DE BREVET EUROPEEN

(11) **EP 0 711 779 B1**
(45) Date de publication et mention de la délivrance du brevet: **28.01.1998**
(21) Numéro de dépôt: 95402228.1
(22) Date de dépôt: 05.10.1995
(51) Int. Cl.: C07F 7/08, C08G 77/34, A61K 7/42

(54) **Nouveaux filtres solaires, compositions cosmétiques photoprotectrices les contenant et utilisations**
Sonnenfilter, diese enthaltende kosmetischen Sonnenschutzzusammensetzungen und deren Verwendung
Sunscreens, cosmetic sunscreen compositions and uses thereof

(30) Priorité: 08.11.1994 FR 9413395
(43) Date de publication de la demande: 15.05.1996
(73) Titulaire: L'OREAL, 75008 Paris (FR)
(72) Inventeur: Richard, Hervé, F-93420 Villepinte (FR); Leduc, Madeleine, Résidence "Les Chèvrefeuilles", F-75011 Paris (FR); Lagrange, Alain, F-77700 Couvray (FR)
(74) Mandataire: Andral, Christophe André Louis

(56) Documents cités:
- EP-A- 0 388 218
- EP-A- 0 392 883
- EP-A- 0 478 284
- WO-A-92/19625
- WO-A-93/04665
- WO-A-93/10745
- WO-A-94/06404
- WO-A-94/10588
- US-A- 5 164 462

## Description

La présente invention concerne de nouveaux composés du type diorganosiloxanes, à chaines courtes, linéaires ou cycliques, ou du type triorganosilanes, présentant pour caractéristique commune d'avoir tous au moins un motif benzotriazole à fonction acrylate ou acrylamide, ces composés étant plus particulièrement utilisables à titre de filtres organiques solaires dans des compositions cosmétiques destinées à la protection de la peau et des cheveux contre le rayonnement ultraviolet. L'invention concerne également l'utilisation desdits composés dans l'application cosmétique susmentionnée, ainsi que les compositions cosmétiques à propriétés améliorées les contenant.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination d'UV-B, provoquent des érythèmes et des brûlures cutanées qui peuvent nuire au développement du bronzage naturel ; il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Il est donc souhaitable de filtrer aussi le rayonnement UV-A.

De nombreux composés destinées à la photoprotection (UV-A et/ou UV-B) de la peau ont été proposées à ce jour.

La plupart d'entre eux sont des composés aromatiques présentant une absorption des rayons UV dans la zone comprise entre 280 et 315 nm, ou dans la zone comprise entre 315 et 400 nm ou bien encore dans l'ensemble de ces deux zones. Ils sont le plus souvent formulés dans des compositions antisolaires qui se présentent sous la forme d'une émulsion de type huile-dans-eau (c'est à dire un support cosmétiquement acceptable constitué d'une phase continue dispersante aqueuse et d'une phase discontinue dispersée huileuse) et qui contiennent donc, à des concentrations diverses, un ou plusieurs filtres organiques classiques à fonction aromatique, lipophiles et/ou hydrophiles, capables d'absorber selectivement les rayonnements UV nocifs, ces filtres (et leurs quantités) étant sélectionnés en fonction de l'indice de protection recherché (l'indice de protection (IP) s'exprimant mathématiquement par le rapport du temps d'irradiation nécessaire pour atteindre le seuil érythématogène avec le filtre UV au temps nécessaire pour atteindre le seuil érythématogène sans filtre UV).

Outre leur pouvoir filtrant, ces composés à activité anti-UV doivent également présenter de bonnes propriétés cosmétiques dans les compositions qui les contiennent, une bonne solubilité dans les solvants usuels et en particulier les corps gras tels que les huiles et les graisses, ainsi qu'une bonne résistance à l'eau et à la transpiration (rémanence).

Parmi tous les composés aromatiques qui ont été préconisés à cet effet, on peut citer notamment les dérivés de l'acide p- aminobenzoïque, les dérivés du benzylidènecamphre, les dérivés de l'acide cinnamique et les dérivés du benzotriazole. Cependant, certaines de ces substances ne présentent pas toutes les propriétés requises pour une utilisation convenable comme filtres UV dans les compositions antisolaires. En particulier, leur pouvoir filtrant intrinsèque peut être insuffisant, leur solubilité dans les différents type de formulations utilisés en matière de protection solaire n'est pas toujours suffisamment bonne (liposolubilité notamment), elles peuvent ne pas posséder une stabilité suffisante à la lumière (photostabilité) et elles peuvent également présenter une mauvaise résistance à l'eau et à la sueur. Il est également souhaitable que ces substances filtrantes ne pénètrent pas dans la peau.

Ainsi, dans le cas plus particulier des substances filtres de type benzotriazole, on a cherché à obtenir des produits à propriétés améliorées, en particulier au niveau de leur liposolubilité et de leur caractère cosmétique, en venant fixer par greffage (hydrosilylation) le groupement filtrant benzotriazole sur une chaine macromoléculaire de type silicone (organopolysiloxane). Cette technique, décrite dans la demande de brevet EP 0 392 883 au nom de la Demanderesse, conduit certes à des composés intéressants (ces produits sont connus sous le nom général de "silicones filtres"), mais le caractère liposoluble de ces derniers peut encore apparaitre comme insuffisant et, de plus, afin d'obtenir des propriétés filtrantes satisfaisantes avec ce type de produits, il est souvent nécessaire de mettre en oeuvre des quantités relativement importantes de ces polymères filtres, ce qui se traduit par de mauvaises propriétés cosmétiques au niveau des formulations qui les contiennent.

Il est connu du document WO94/06404 des compositions comprenant du 4-(tert.-butyl) 4'-méthoxy dibenzoylméthane et un polymère filtrant du type silicone benzotriazole.

La présente invention vise à résoudre les problèmes ci-dessus en proposant de nouveaux composés, de type silicones filtres, à motif benzotriazole, qui présentent des propriétés améliorées, notamment au niveau de leur solubilité dans les corps gras et de leurs propriétés cosmétiques.

Plus précisemment encore, il a été trouvé, selon la présente invention, qu'en associant, notamment par réaction d'hydrosylilation, un ou plusieurs dérivés de benzotriazole particuliers, à savoir plus présisément des acrylates ou des acrylamides de benzotriazoles, avec une chaine silicone particulière, linéaire ou cyclique, ou un silane particulier, il était possible d'aboutir à de nouveaux composés du type silicones filtres obviant aux inconvénients des silicones filtres de l'art antérieur, ces nouveaux composés présentant en particulier de très bonnes propriétés filtrantes, tant dans l'UV-A que dans l'UV-B, une très bonne solubilité dans les solvants organiques usuels et notamment les corps gras tels que les huiles, ainsi que d'excellentes propriétés cosmétiques, les rendant particulièrement appropriés à une utilisation comme filtres solaires dans des, ou pour la préparation de, compositions cosmétiques destinées à la protection de la peau et/ou des cheveux contre le rayonnement ultraviolet.

La présente invention a ainsi pour premier objet de nouveaux composés qui sont caractérisés par le fait qu'ils répondent à l'une des formules (1) à (3) suivantes : 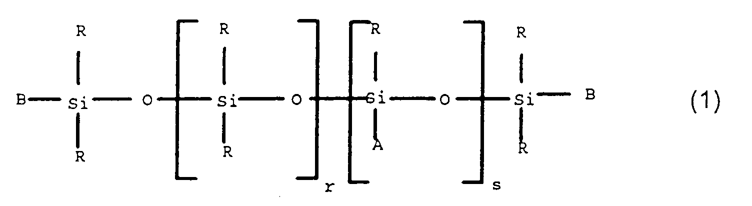 ou 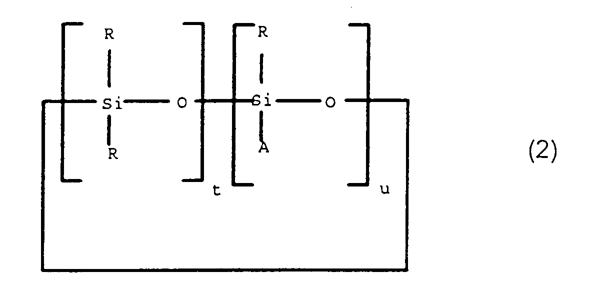 ou

A-Si(R')₃ (3)

formules (1) à (3) dans lesquelles :
- R, identiques ou différents, sont choisis parmi les radicaux alkyles en C₁-C₁₀, phényle et trifluoro-3,3,3 propyle, au moins 80% en nombre des radicaux R étant méthyle,
- B, identiques ou différents, sont choisis parmi les radicaux R ci-dessus et le radical A défini ci-après,
- R', identiques ou différents, sont choisis parmi les radicaux alkyles en C₁-C₈, ou phényle,
- r est un nombre entier compris entre 0 et 50 inclusivement, et s est un nombre entier compris entre 0 et 20 inclusivement, avec la condition que si s est nul alors au moins l'un des deux symboles B désigne A,
- u est un nombre entier compris entre 1 et 6 inclusivement, et t est un nombre entier compris entre 0 et 10 inclusivement, étant entendu que t + u est égal ou supérieur à 3,
- et le symbole A désigne un radical monovalent lié directement à un atome de silicium, et qui répond à la formule (4) suivante : 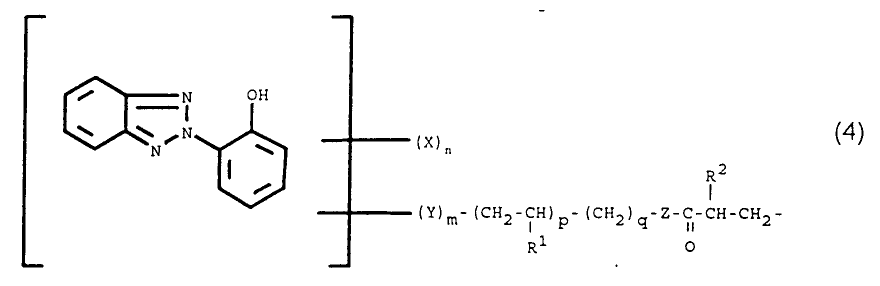
   formule (4) dans laquelle :
   - n est un nombre entier compris entre 0 et 3 inclusivement et X, identiques ou différents, sont choisis parmi les radicaux alkyles en C₁-C₈, les halogènes et les radicaux alkoxy en C₁-C₄,
   - m est 0 ou 1, et Y représente -O-, -NH-, -COO-, -O(CH₂)ᵥ-COO- ou -(CH₂)_{w}-OCONH-, v et w étant des nombres entiers compris entre 0 et 12 inclusivement,
   - p est 0 ou 1,
   - q est un nombre entier compris entre 0 et 12 inclusivement,
   - Z représente -O- ou -NH-,
   - R¹ représente l'hydrogène ou un radical alkyle en C₁-C₄,
   - R² représente l'hydrogène ou un radical méthyle.

Dans les formules (1) à (3) ci-dessus, A représente donc le groupement dérivé du benzotriazole qui, après fixation sur la chaine courte siliconée de départ ou sur le silane de départ, confère aux composés de type diorganosiloxanes linéaires (formule (1)) ou cycliques (formule (2)) ou de type triorganosilanes (formule (3)) des propriétés absorbantes à la fois vis à vis des UV-A et des UV-B. Comme indiqué précédemment, et comme cela ressort de la définition de la formule (4) donnée ci-dessus, ce groupement présente nécessairement soit une fonction acrylate (Z = O) soit une fonction acrylamide (Z = NH) qui est apportée par le chainon qui assure l'accrochage du benzotriazole à la chaine silicone ou au silane.

Comme cela ressort de la formule (4) donnée ci-dessus, l'accrochage du chainon -(Y)ₘ-(CH₂-CHR¹)ₚ-(CH₂)_{q}-Z-CO-CHR²-CH₂- sur le motif benzotriazole, qui assure donc le raccordement dudit motif benzotriazole à l'atome de silicium de la chaine siliconée ou du silane, peut, selon la présente invention, se faire dans toutes les positions disponibles offertes par les deux noyaux aromatiques du benzotriazole :

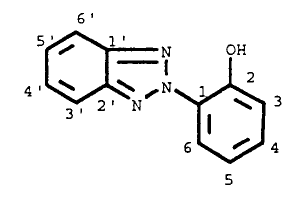

De préférence, cet accrochage se fait en position 3, 4, 5 (noyau aromatique portant la fonction hydroxy) ou 4' (noyau benzénique adjacent le cycle triazolé), et encore plus préférentiellement en position 3, 4 ou 5.

De même, l'accrochage du motif subsituant X peut se faire dans toutes les autres positions disponibles au sein du benzotriazole. Toutefois, de préférence, cet accrochage se fait en position 3, 4, 4', 5 et/ou 6.

Dans les formules (1) à (3) ci-dessus, les radicaux alkyle peuvent être linéaires ou ramifiés et choisis notamment au sein des radicaux méthyle, éthyle, n-propyle, isopropyle, n-butyle, isobutyle, ter.-butyle, n-amyle, isoamyle, néopentyle, n-hexyle, n-heptyle, n-octyle, éthyl-2 hexyle et ter.-octyle. Les radicaux alkyle R, R' et B préférés selon l'invention sont les radicaux méthyle, éthyle, propyle, n-butyle, n-octyle et éthyl-2 hexyle. Encore plus préférentiellement, les radicaux R, R' et B sont tous des radicaux méthyle.

Parmi les composés de formules (1) à (3) ci-dessus, on préfère mettre en oeuvre ceux répondant à la formule (1) ou à la formule (2), c'est à dire des diorganosiloxanes à chaine courte, linéaire ou cyclique.

Parmi les diorganosiloxanes linéaires ou cycliques rentrant dans le cadre de la présente invention, on préfère plus particulièrement les dérivés statistiques ou bien définis à blocs présentant au moins l'une, et encore plus préférentiellement l'ensemble, des caractéristiques suivantes :
- R est alkyle et encore plus préférentiellement est méthyle,
- B est alkyle et encore plus préférentiellement méthyle (cas des composés linéaires de formule (1)),
- r est compris entre 0 et 3 inclusivement ; s est compris entre 0 et 3 inclusivement (cas des composés linéaires de formule (1)),
- t+u est compris entre 3 et 5 (cas des composés cycliques de formule (2)),
- n est non nul, et de préférence égal à 1 ou 2, et X est alors choisi parmi méthyle, ter.-butyle ou alcoxy en C₁-C₄, notamment méthoxy,
- m est non nul et Y représente -O- ou -NH-
- p est non nul et R¹ est H
- q est compris entre 0 et 3 inclusivement
- Z représente -O- ou -NH-

Pour préparer les filtres siliconés de formule (1) et (2), on peut, selon une première méthode (méthode 1), procéder classiquement en mettant en oeuvre une réaction d'hydrosylilation du type 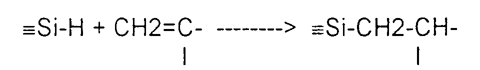 à partir de la silicone correspondante dans laquelle, par exemple, tous les radicaux A sont des atomes d'hydrogène. Cette silicone de départ est dénommée par la suite dérivé à SiH ; les groupes SiH peuvent être présents dans la chaîne et/ou aux extrémités de la chaîne silicone. Ces dérivés à SiH sont des produits bien connus dans l'industrie des silicones et sont généralement disponibles dans le commerce. Ils sont par exemple décrits dans Les brevets américains US-A- 3 220 972, US-A- 3 697 473 et US-A- 4 340 709.
Ce dérivé à SiH peut être donc représenté soit par la formule (1bis) suivante : 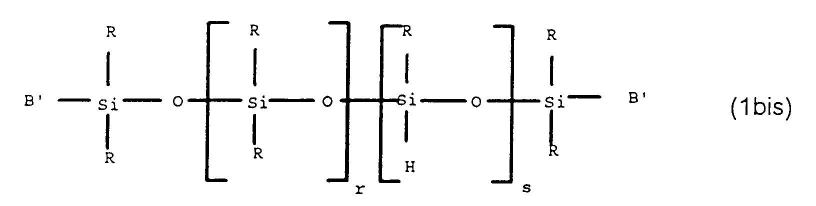 dans laquelle R, r et s ont la signification donnée ci-dessus pour la formule (1) et les radicaux B', identiques ou différents, sont choisis parmi les radicaux R et un atome d'hydrogène,
soit par la formule (2bis) suivante : 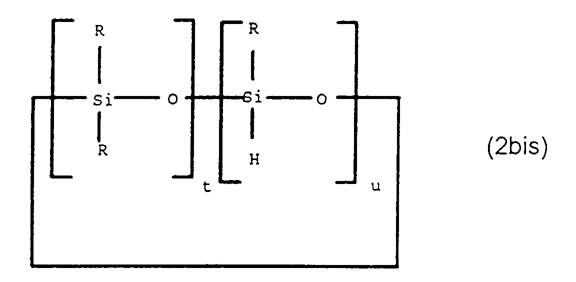 dans laquelle R, t et u ont la signification donnée ci-dessus pour la formule (2).

Sur ce dérivé à SiH de formules (1bis) ou (2bis), on effectue donc une réaction d'hydrosilylation classique, opérée en présence d'une quantité catalytiquement efficace d'un catalyseur au platine, sur un dérivé organique de benzotriazole de formule (4bis) suivante : 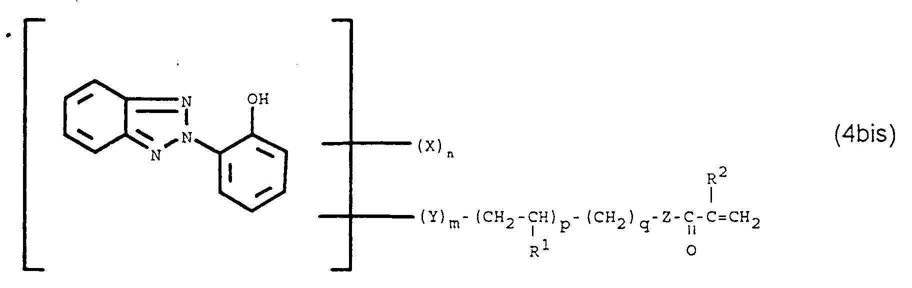 dans laquelle X, Y, Z, R¹, R², n, m p et q ont la signification donnée ci-dessus pour la formule (4).

Des procédés convenant à la préparation des produits de formule (4bis) ci-dessus sont notamment décrits dans les brevets US- 4 316 033 et US- 4 328 346. Par ailleurs, certains de ces produits sont disponibles commercialement. En particulier, le 2-(2'-hydroxy-5-méthacrylyloxyéthylphényl)-2H-benzotriazole est vendu sous le nom commercial de "Norbloc 7966®" par la Société NORAMCO.
En outre, les détails des conditions opératoires à suivre pour conduire la réaction d'hydrosylilation entre les composés de formule (1bis) ou (2bis) ci-dessus avec le composé de formule (4bis) ci-dessus sont donnés dans la demande de brevet EP- 0 392 883 précitée, dont l'enseignement est, à cet égard, totalement inclus à titre de référence dans la présente description.

Concernant la préparation des filtres de type triorganosilanes de formule (3) donnée précédemment, on peut procéder comme indiqué ci-dessus, toujours par réaction d'hydrosylilation, entre un silane de départ de formule (R')₃Si-H (formule (3bis), dans laquelle R' a la même signification que pour le composé de formule (3), et un dérivé organique de benzotriazole de formule (4bis) définie ci-avant.

Des composés de formule (4bis) rentrant particulièrement bien dans le cadre de la présente invention sont notamment les :
a) 2-(2'-hydroxy-5'-méthacrylyloxy éthyl phényl)-2H-benzotriazole
b) 5-méthoxy-2-(2'-hydroxy-4'-méthacrylyloxy phényl)-2H-benzotriazole
c) 5-méthoxy-2-(2'-hydroxy-3'-terbutyl-5'-méthacrylyloxy éthyl phényl)-2H-benzotriazole
d) 5-méthyl-2-(2'-hydroxy-5'-méthacrylyloxy éthyl oxy phényl)-2H-benzotriazole
e) 5-méthacrylyloxy éthyl-2-(2'-hydroxy-3',5'-diterbutyl phényl)-2H-benzotriazole.

Une autre voie de synthèse possible (méthode 2) convenant à la préparation des filtres siliconés de formule (1) et (2) consiste à partir des dérivés correspondant à la formule (1) ou (2) dans laquelle tous les radicaux A sont remplacés par le radical de formule (5) : 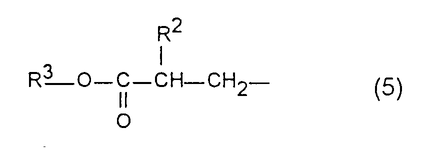 dans laquelle R² a la même signification que ci-avant et R³ est un atome d'hydrogène ou un radical méthyle ou éthyle.

Sur ce dérivé siloxane carboxylique, on fait alors réagir l'alcool, le phénol ou l'amine correspondant à la formule (4ter) : 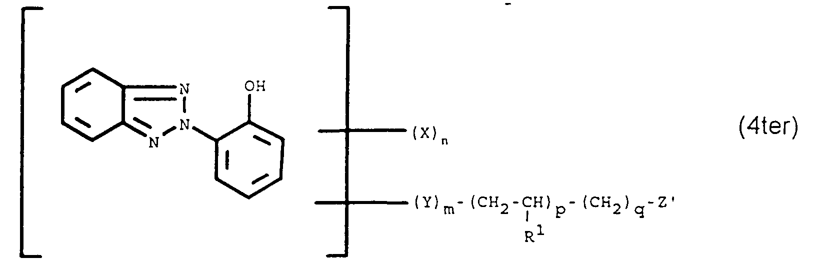 dans laquelle X, Y, R¹, n, m, p et q ont la signification donnée ci-avant pour la formule (4) et Z' représente -OH ou -NH₂.

Par rapport aux silicones filtres de l'art antérieur telles que décrites dans la demande de brevet EP 0 392 883 précitée, les silicones filtres selon l'invention présentent donc une ou plusieurs différences structurelles essentielles, à l'origine de leurs propriétés remarquables : les chaines siliconées, sur lesquelles sont greffés le ou les motifs benzotriazoles, sont tout d'abord beaucoup plus courtes ; ensuite, le motif dérivé du benzotriazole porte toujours au moins une fonction acrylate ou acrylamide.

Comme indiqué précédemment, les composés de formules (1) à (3) ci-dessus présentent un excellent pouvoir filtrant intrinsèque à l'égard du rayonnement ultraviolet UV-A et UV-B. En outre, de par leur caractère fortement liposoluble, les composés de formule (1) à (3) ci-dessus peuvent être utilisés à de grandes concentrations, ce qui confère aux compositions finales des indices de protection très élevés ; par ailleurs, ils se répartissent uniformément dans les supports cosmétiques classiques contenant au moins une phase grasse ou un solvant organique cosmétiquement acceptable et peuvent être ainsi appliqués sur la peau ou les cheveux pour constituer un film protecteur efficace. Enfin, leurs propriétés cosmétiques sont très bonnes, à savoir en particulier que ces produits, par rapport aux silicones filtres de l'art antérieur, sont moins collantes et apportent plus de douceur.

La présente invention a donc aussi pour objet une composition cosmétique comprenant, dans un support cosmétiquement acceptable contenant de préférence au moins une phase grasse ou un solvant organique, une quantité efficace d'au moins un composé de formules (1) à (3) définies ci-avant.

Les composés de formules (1) à (3) sont généralement présents dans des proportions comprises entre 0,1 % et 20 % en poids, de préférence entre 0,5 % et 10 % en poids, par rapport au poids total de la composition.

La composition cosmétique de l'invention peut être utilisée comme composition protectrice de l'épiderme humain ou des cheveux contre les rayons ultraviolets, comme composition antisolaire ou comme produit de maquillage.

Cette composition peut se présenter en particulier sous forme de lotion, de lotion épaisse, de gel, de crème, de lait, de poudre, de bâtonnet solide et eventuellement être conditionnée en aérosol et se présenter sous forme de mousse ou de spray.

Elle peut contenir les adjuvants cosmétiques habituellement utilisés dans le domaine, tels que des corps gras, des solvants organiques, des silicones, des épaississants, des adoucissants, des filtres solaires complémentaires, des agents anti-mousses, des agents hydratants, des parfums, des conservateurs, des tensio-actifs, des charges, des sequestrants, des polymères anioniques, cationiques, non ioniques ou amphotères ou leurs mélanges, des propulseurs, des agents alcalinisants ou acidifiants, des colorants, des pigments ou nanopigments en particulier ceux destinés à assurer un effet photoprotecteur complémentaire par blocage physique du rayonnement ultraviolet, ou tout autre ingrédient habituellement utilisé en cosmétique, en particulier pour la fabrication des compositions antisolaires.

Parmi les solvants organiques, on peut citer les alcools et polyols inférieurs tels que l'éthanol, l'isopropanol, le propylèneglycol, la glycérine et le sorbitol.

Les corps gras peuvent être constitués par une huile ou par une cire ou leurs mélanges, des acides gras, des esters d'acides gras, des alcools gras, la vaseline, la paraffine, la lanoline, la lanolinehydrogénée, la lanoline acétylée. Les huiles peuvent être choisies parmi les huiles animales, végétales, minérales ou de synthèse, et notamment l'huile de palme hydrogénée, l'huile de ricin hydrogénée, l'huile de vaseline, l'huile de paraffine, l'huile de Purcellin, les huiles de silicones, volatiles ou non, et les isoparaffines.

Lorsque la composition cosmétique selon l'invention est plus particulièrement destinée à la protection de l'épiderme humain contre les rayons UV ou comme composition antisolaire, elle peut se présenter sous forme de suspension ou de dispersion dans des solvants ou des corps gras, ou encore sous forme d'émulsion (notamment de type H/E ou E/H, mais de préférence H/E) telle qu'une crème ou un lait, de dispersion vésiculaire, sous forme de pommade, de gel, de bâtonnet solide ou de mousse aérosol. Les émulsions peuvent contenir en outre des agents tensio-actifs anioniques, non ioniques, cationiques ou amphotères.

Lorsque la composition cosmétique selon l'invention est utilisée pour la protection des cheveux, elle peut se présenter sous forme de shampooing, de lotion, de gel ou composition à rincer, à appliquer avant ou après shampooing, avant ou après coloration ou décoloration, avant, pendant ou après permanente ou défrisage, de lotion ou gel coiffant ou traitant, de lotion ou gel pour le brushing ou la mise en plis, de laque pour cheveux, de composition de permanente ou de défrisage, de coloration ou décoloration des cheveux.

Lorsque la composition cosmétique selon l'invention est utilisée comme produit de maquillage des cils, des sourcils, de la peau ou des cheveux, tels que crème de traitement de l'épiderme, fond de teint, bâton de rouge à lèvres, fards à paupière, fards à joues, ligneur encore appelé "eye-liner", mascara, gel colorant, elle peut se présenter sous forme solide ou pâteuse, anhydre ou aqueuse, comme des émulsions huile-dans-eau ou eau-dans-huile, des suspensions ou encore des gels.

L'invention a aussi pour objet un procédé de protection de la peau et cheveux contre le rayonnement ultraviolet, en particulier le rayonnement solaire, qui consiste à appliquer sur la peau ou les cheveux une quantité efficace de la composition cosmétique définie ci-dessus, ou d'un composé de formule (1), (2) ou (3) tels que définis ci-avant.

Les exemples qui suivent illustrent l'invention sans toutefois en limiter la portée.

### EXEMPLE 1

Cet exemple illuste la préparation (selon la méthode 1) d'un composé conforme à l'invention de formule : 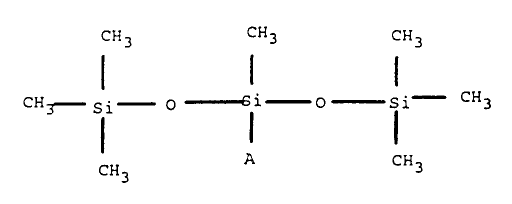 dans laquelle A représente : 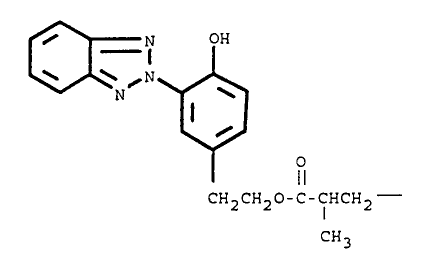 (ce produit correspond à un composé de formule (1) pour lequel R = B = CH₃ ; r = 0 ; s = 1 ; n = 0 ; m = 0 ; p = 0 ; q = 2 ; Z = O ; R² = CH₃)
Dans un réacteur, on charge 30 g de 2-(2'-hydroxy-5-méthacrylyloxyéthylphényl)-2H-benzotriazole ("Norbloc 7966®") et 50 ml de toluène. Le mélange est porté à 80°C sous azote. On ajoute le catalyseur d'hydrosylilation (complexe à 3-3,5 % de Pt dans du cyclovinylméthylsiloxane de Hüls Petrarch PC085 : 100 µl) puis 24,5 g d'heptaméthyltrisiloxane. Après 67 heures à 80°C sous azote avec ajouts de 50 µl de catalyseur toutes les 12 heures, on concentre le milieu réactionnel et on effectue ensuite une chromatographie sur silice sous pression (éluant : heptane avec gradient 0-50% de CH2Cl2). On récupère alors 5,1 g du produit final désiré, qui se présente sous la forme d'une huile jaune pâle.

Les caractéristiques en absorption UV (mesurées dans l'éthanol) de ce produit sont les suivantes :

| | |
|---|---|
| λₘₐₓ : 337 nm | εₘₐₓ : 17 500 |
| λₘₐₓ : 298 nm | εₘₐₓ : 14 550 |

Ce produit peut donc être utilisé très efficacement à titre de filtre solaire actif dans l'UV-A et l'UV-B.

### EXEMPLE 2

Cet exemple illuste la préparation, mais cette fois selon la méthode 2, du même composé que celui de l'exemple 1 précédent.

### a) Première étape :

Dans un réacteur, on porte à 80°C sous azote 34,24 g de méthacrylate d'éthyle contenant une pointe de spatule de 4-hydroxy anisole. On y ajoute le catalyseur d'hydrosilylation (complexe à 3-3,5 % de Pt dans du cyclovinylméthylsiloxane de Hüls Petrarch PC085 : 200 µl) puis, goutte à goutte et en 30 minutes, 73,42 g d'heptaméthyltrisiloxane. Après 3 heures d'agitation à 80°C sous azote, on concentre le milieu, on évapore l'acrylate et le siloxane en excès et récupère ainsi une huile jaune pâle. Cette huile est ensuite distillée sous vide. La fraction distillant à 48-52°C sous 20 mmHg correspond au dérivé recherché de formule (1) dans laquelle : R = B = CH₃ ; r = 0 ; s = 1 ; et A est le radical de formule (5) avec R² = méthyle et R³ = éthyle.

### b) Deuxième étape :

Dans un réacteur muni d'un Dean Stark, on charge 340 ml de toluène, 20 g du dérivé préparé à l'étape précédente et 12,7 g de 2-(2'-hydroxy-5'-hydroxy éthyl phényl)-2H-benzotriazole (c'est à dire un dérivé de formule (4ter) dans laquelle
n= 0 ; m = 0 ; p = 1 ; R¹ = H ; q = 0 et Z' = OH). On ajoute 0,5 g d'acide p-toluène sulfonique et on chauffe au reflux pendant 20 heures tout en éliminant l'éthanol formé. On concentre le milieu réactionnel et on effectue ensuite une chromatographie sur silice sous pression (éluant : heptane avec gradient 0-50% de CH2Cl2). On récupère ainsi 9,8 g du produit final désiré, et correspondant au produit préparé à l'exemple 1.

### EXEMPLE 3

On illustre ici une formulation concrète de composition cosmétique antisolaire conforme à l'invention, à savoir une crème antisolaire.

| | |
|---|---|
| - Composé de l'exemple 1 | 5 g |
| - Mélange d'alcool cétylstéarylique et d'alcool cétylstéarylique oxyéthyléné à 33 moles d'OE ("SINNOVAX AO" de chez HENKEL) | 7 g |
| - Mélange de mono et distéarate de glycérol non autoémulsifiable | 2 g |
| - Alcool cétylique | 1,5 g |
| - Benzoate d'alcools en C₁₂-C₁₅ ("FINSOLV TN" de chez WITCO) | 20 g |
| - Polydiméthylsiloxane | 1,5 g |
| - Glycérine | 17,5 g |
| - Parfum, conservateur qs | |
| - Eau qsp | 100 g |

Cette crème est préparée selon les techniques classiques de préparation d'émulsions en dissolvant le filtre dans la phase grasse contenant les émulsionnants, en chauffant cette phase grasse vers 70-80°C et en ajoutant, sous vive agitation, l'eau chauffée à la même température. On maintient l'agitation pendant 10 à 15 minutes, puis on laisse refroidir sous agitation modérée, et vers 40 °C on ajoute enfin parfum et conservateur.

## Revendications

1. composés de formules : 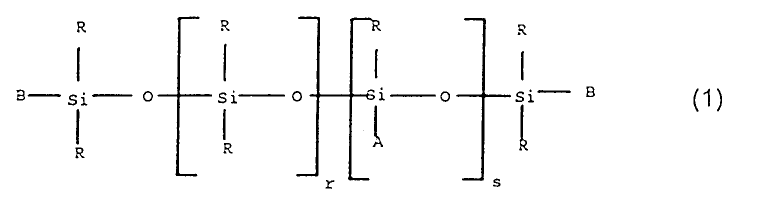 ou 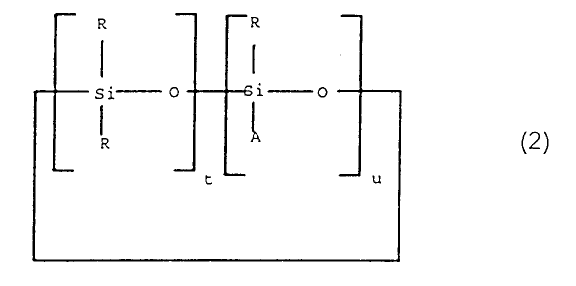 ou
A-Si(R')₃ (3)
formules (1) à (3) dans lesquelles :
- R, identiques ou différents, sont choisis parmi les radicaux alkyles en C₁-C₁₀, phényle et trifluoro-3,3,3 propyle, au moins 80% en nombre des radicaux R étant méthyle,
- B, identiques ou différents, sont choisis parmi les radicaux R ci-dessus et le radical A défini ci-après,
- R', identiques ou différents, sont choisis parmi les radicaux alkyles en C₁-C₈, ou phényle,
- r est un nombre entier compris entre 0 et 50 inclusivement, et s est un nombre entier compris entre 0 et 20 inclusivement, avec la condition que si s est nul alors au moins l'un des deux symboles B désigne A,
- u est un nombre entier compris entre 1 et 6 inclusivement, et t est un nombre entier compris entre 0 et 10 inclusivement, étant entendu que t + u est égal ou supérieur à 3,
- et le symbole A désigne un radical monovalent lié directement à un atome de silicium, et qui répond à la formule (4) suivante : 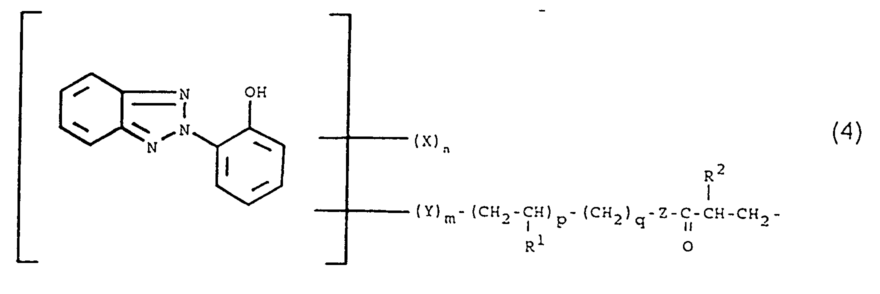
formule (4) dans laquelle :
- n est un nombre entier compris entre 0 et 3 inclusivement et X, identiques ou différents, sont choisis parmi les radicaux alkyles en C₁-C₈, les halogènes et les radicaux alkoxy en C₁-C₄,
- m est 0 ou 1, et Y représente -O-, -NH-, -COO-, -O(CH₂)ᵥCOO- ou -(CH₂)_{w}-OCONH-, v et w étant des nombres entiers compris entre 0 et 12 inclusivement,
- p est 0 ou 1,
- q est un nombre entier compris entre 0 et 12 inclusivement,
- Z représente -O- ou -NH-,
- R¹ représente l'hydrogène ou un radical alkyle en C₁-C₄,
- R² représente l'hydrogène ou un radical méthyle.

2. x composés selon la revendication 1, répondant à la formule (1) ou à la formule (2), caractérisés par le fait que les radicaux R sont des radicaux alkyle.

3. composés selon la revendication 2, caractérisés par le fait que les radicaux R sont des radicaux méthyle, éthyle, propyle, n-butyle, n-octyle ou éthyl-2 hexyle.

4. composés selon la revendication 3, caractérisés par le fait que les radicaux R sont des radicaux méthyle.

5. composés selon l'une quelconque des revendications précédentes, répondant à la formule (1), caractérisés par le fait que les radicaux B sont des radicaux alkyle.

6. composés selon la revendication 5, caractérisés par le fait que les radicaux B sont des radicaux méthyle, éthyle, propyle, n-butyle, n-octyle ou éthyl-2 hexyle.

7. composés selon la revendication 6, caractérisés par le fait que les radicaux B sont radicaux méthyle.

8. composés selon l'une quelconque des revendications précédentes, répondant à la formule (1), caractérisés par le fait que r est compris entre 0 et 3 inclusivement, et s est compris entre 0 et 3 inclusivement.

9. composés selon l'une quelconque des revendications 1 à 4, répondant à la formule (2), caractérisés par le fait que t + u est compris entre 3 et 5 inclusivement.

10. composés selon la revendication 1, répondant à la formule (3), caractérisés par le fait que les radicaux R' sont des radicaux alkyle choisis parmi les radicaux méthyle, éthyle, propyle, n-butyle, n-octyle ou éthyl-2 hexyle.

11. composés selon la revendication 10, caractérisés par le fait que les radicaux R' sont des radicaux méthyle.

12. composés selon l'une quelconque des revendications précédentes, caractérisés par le fait que n est non nul, de préférence égal à 1 ou 2, et X est choisi parmi méthyle, ter.-butyle, alcoxy en C₁-C₄, en particulier méthoxy.

13. composés selon l'une quelconque des revendications précédentes, caractérisés par le fait que m est non nul et Y représente -O- ou - NH-.

14. composés selon l'une quelconque des revendications précédentes, caractérisés par le fait que p est non nul et R¹ est l'hydrogène.

15. composés selon l'une quelconque des revendications précédentes, caractérisés par le fait que q est compris entre 0 et 3 inclusivement.

16. composés selon l'une quelconque des revendications précédentes, caractérisés par le fait que l'accrochage du chainon -(Y)ₘ-(CH₂-CHR¹)ₚ-(CH₂)_{q}-Z-CO-CHR²-CH₂- sur le motif benzotriazole se fait en position 3, 4, 4' ou 5 de ce dernier.

17. composés selon la revendication 16, caractérisés par le fait que ledit accrochage se fait en position 3, 4 ou 5.

18. composés selon l'une quelconque des revendications précédentes, caractérisés par le fait que l'accrochage du substituant X sur le motif benzotriazole se fait en position(s) 3, 4, 4', 5 et/ou 6 de ce dernier.

19. Utilisation non thérapeutique des composés de formule (1)-(3) tels que définis à l'une quelconque des revendications 1 à 18, à titre de filtres solaires actifs dans le domaine des UV-A et UV-B.

20. Composition cosmétique, en particulier pour la filtration des rayons ultraviolets, caractérisée par le fait qu'elle comprend, dans un support cosmétiquement acceptable, une quantité efficace d'au moins un des composés définis à l'une quelconque des revendications 1 à 18.

21. Composition cosmétique selon la revendication 20, caractérisée par le fait que ledit support cosmétiquement acceptable contient au moins une phase grasse ou un solvant organique.

22. Composition cosmétique selon la revendication 21, caractérisée par le fait que ledit support se présente sous la forme d'une émulsion de type huile-dans-eau ou eau-dans-huile, de préférence huile-dans-eau.

23. Composition cosmétique selon l'une quelconque des revendications 20 à 22, caractérisée par le fait que la teneur en composé(s) filtrant(s) est comprise entre 0,1 et 20 % en poids par rapport au poids total de la composition.

24. Composition cosmétique selon la revendication 23, caractérisée par le fait que ladite teneur est comprise entre entre 0,5 et 10 % en poids.

25. Procédé non thérapeutique de protection de la peau et/ou des cheveux contre le rayonnement ultraviolet, en particulier le rayonnement solaire, caractérisé par le fait qu'il consiste à appliquer sur la peau et/ou les cheveux une quantité efficace d'au moins un composé ou d'au moins une composition tels que définis à l'une quelconque des revendications précédentes.

## Claims

1. Compounds of formulae: 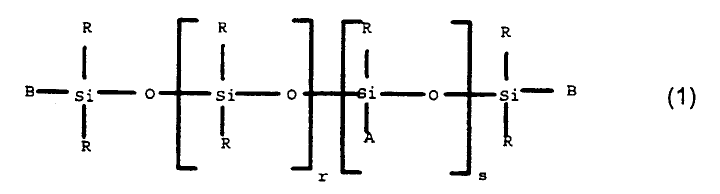 or 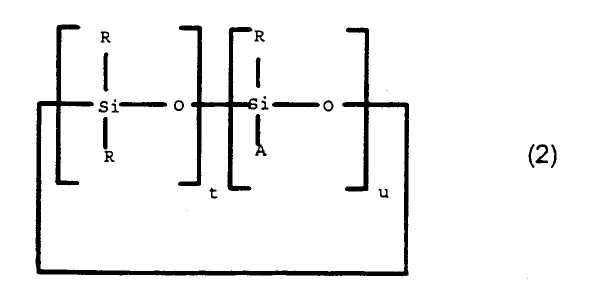 or
A-Si(R')₃ (3)
in which formulae (1) to (3):
- the groups R, which may be identical or different, are chosen from C₁-C₁₀ alkyl, phenyl and 3,3,3-trifluoropropyl radicals, at least 80 %, in numerical terms, of the radicals R being methyl,
- the groups B, which may be identical or different, are chosen from the above radicals R and the radical A defined below,
- the groups R', which may be identical or different, are chosen from C₁-C₈ alkyl radicals, or phenyl radicals,
- r is an integer between 0 and 50 inclusively, and s is an integer between 0 and 20 inclusively, with the condition that if s is zero then at least one of the two symbols B denotes A,
- u is an integer between 1 and 6 inclusively, and t is an integer between 0 and 10 inclusively, it being understood that t + u is equal to or greater than 3,
- and the symbol A denotes a monovalent radical directly attached to a silicon atom and which corresponds to the following formula (4): 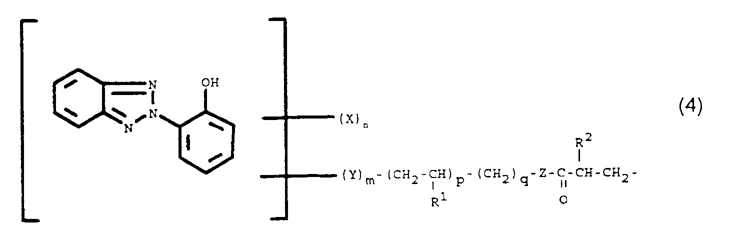 in which formula (4):
- n is an integer between 0 and 3 inclusively and the groups X, which may be identical or different, are chosen from C₁-C₈ alkyl radicals, halogens and C₁-C₄ alkoxy radicals,
- m is 0 or 1, and Y represents -O-, -NH-, -COO-, -O(CH₂)ᵥ-COO- or -(CH₂)_{w}-OCONH-, v and w being integers between 0 and 12 inclusively,
- p is 0 or 1,
- q is an integer between 0 and 12 inclusively,
- Z represents -O- or -NH-,
- R¹ represents hydrogen or a C₁-C₄ alkyl radical,
- R² represents hydrogen or a methyl radical.

2. Compounds according to Claim 1, corresponding to formula (1) or to formula (2), characterized in that the radicals R are alkyl radicals.

3. Compounds according to Claim 2, characterized in that the radicals R are methyl, ethyl, propyl, n-butyl, n-octyl or 2-ethylhexyl radicals.

4. Compounds according to Claim 3, characterized in that the radicals R are methyl radicals.

5. Compounds according to any one of the preceding claims, corresponding to formula (1), characterized in that the radicals B are alkyl radicals.

6. Compounds according to Claim 5, characterized in that the radicals B are methyl, ethyl, propyl, n-butyl, n-octyl or 2-ethylhexyl radicals.

7. Compounds according to Claim 6, characterized in that the radicals B are methyl radicals.

8. Compounds according to any one of the preceding claims, corresponding to formula (1), characterized in that r is between 0 and 3 inclusively, and s is between 0 and 3 inclusively.

9. Compounds according to any one of Claims 1 to 4, corresponding to formula (2), characterized in that t + u is between 3 and 5 inclusively.

10. Compounds according to Claim 1, corresponding to formula (3), characterized in that the radicals R' are alkyl radicals chosen from the methyl, ethyl, propyl, n-butyl, n-octyl and 2-ethylhexyl radicals.

11. Compounds according to Claim 10, characterized in that the radicals R' are methyl radicals.

12. Compounds according to any one of the preceding claims, characterized in that n is non-zero, preferably equal to 1 or 2, and X is chosen from methyl, tert-butyl and C₁-C₄ alkoxy, in particular methoxy.

13. Compounds according to any one of the preceding claims, characterized in that m is non-zero and Y represents -O- or -NH-.

14. Compounds according to any one of the preceding claims, characterized in that p is non-zero and R¹ is hydrogen.

15. Compounds according to any one of the preceding claims, characterized in that q is between 0 and 3 inclusively.

16. Compounds according to any one of the preceding claims, characterized in that the chain unit -(Y)ₘ-(CH₂-CHR¹)ₚ-(CH₂)_{q}-Z-CO-CHR²-CH₂- is anchored onto the benzotriazole unit in position 3, 4, 4' or 5 of the latter.

17. Compounds according to Claim 16, characterized in that the said anchoring takes place in position 3, 4 or 5.

18. Compounds according to any one of the preceding claims, characterized in that the substituent X is anchored onto the benzotriazole unit in position(s) 3, 4, 4', 5 and/or 6 of the latter.

19. Non-therapeutic use of the compounds of formulae (1)-(3) as defined in any one of Claims 1 to 18, as sunscreens which are active in the UV-A and UV-B region.

20. Cosmetic composition, in particular for screening out ultraviolet rays, characterized in that it comprises, in a cosmetically acceptable vehicle, an effective amount of at least one of the compounds defined in any one of Claims 1 to 18.

21. Cosmetic composition according to Claim 20, characterized in that the said cosmetically acceptable vehicle contains at least one fatty phase or one organic solvent.

22. Cosmetic composition according to Claim 21, characterized in that the said vehicle is in the form of an emulsion of oil-in-water or water-in-oil type, preferably of oil-in-water type.

23. Cosmetic composition according to any one of Claims 20 to 22, characterized in that the content of screening compound(s) is between 0.1 and 20 % by weight relative to the total weight of the composition.

24. Cosmetic composition according to Claim 23, characterized in that the said content is between 0.5 and 10 % by weight.

25. Non-therapeutic process for protecting the skin and/or the hair against ultraviolet radiation, in particular solar radiation, characterized in that it consists in applying to the skin and/or the hair an effective amount of at least one compound or of at least one composition as are defined in any one of the preceding claims.

## Patentansprüche

1. Verbindungen der Formeln: 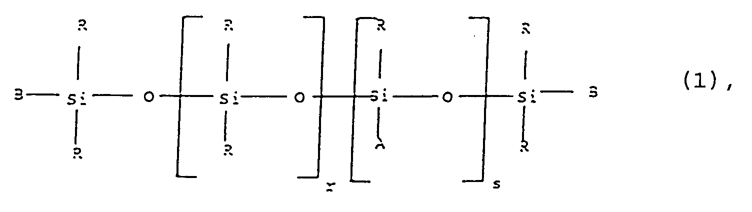 oder 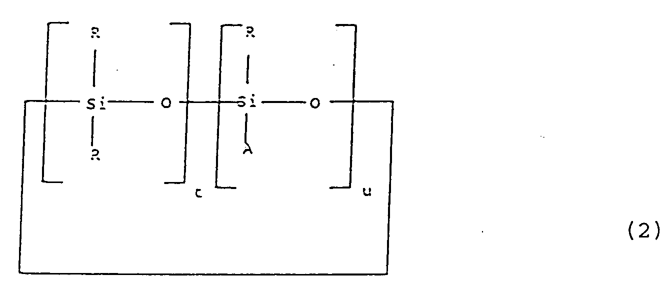 oder
A-Si(R')₃ (3),
wobei in den Formeln (1) bis (3)
- die Gruppen R, die identisch oder voneinander verschieden sind, unter den C₁₋₁₀-Alkylgruppen, Phenyl und 3,3,3-Trifluorpropyl ausgewählt sind, wobei mindestens 80 % der Gruppen R Methyl bedeuten,
- die Gruppen B, die identisch oder voneinander verschieden sind, unter den oben genannten Gruppen R und der nachfolgend definierten Gruppe A ausgewählt sind,
- die Gruppen R', die identisch oder voneinander verschieden sind, unter den C₁₋₈-Alkylgruppen und Phenyl ausgewählt sind,
- r Null oder eine ganze Zahl von 1 bis 50 und s Null oder eine ganze Zahl von 1 bis 20 bedeutet, mit der Maßgabe, daß mindestens eine der beiden Gruppen B A bedeutet, wenn s Null ist,
- u eine ganze Zahl im Bereich von 1 bis 6 und t Null oder eine ganze Zahl im Bereich von 1 bis 10 bedeutet, mit der Maßgabe, daß u + t mindestens 3 ist,
- die Gruppe A eine einwertige Gruppe bedeutet, die direkt an ein Siliciumatom gebunden ist und der folgenden Formel (4) entspricht: 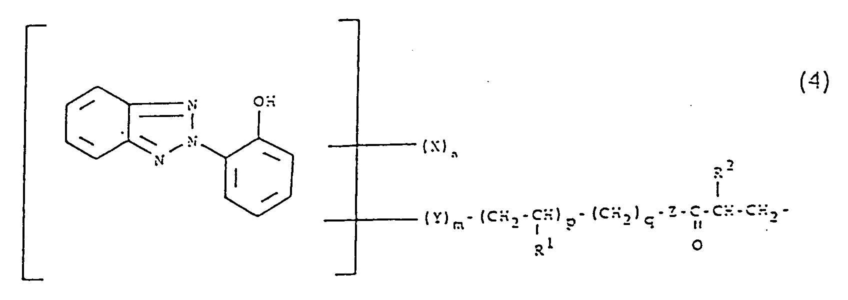 wobei in der Formel (4):
- n Null oder eine ganze Zahl im Bereich von 1 bis 3 bedeutet und die Gruppen X, die identisch oder voneinander verschieden sind, unter C₁₋₈-Alkylgruppen, Halogenen und C₁₋₄-Alkoxygruppen ausgewählt sind,
- m Null oder 1 und Y -O-, -NH-, -COO-, -O(CH₂)ᵥ-COO- oder -(CH₂)_{w}-OCONH- bedeutet, wobei v und w Null oder ganze Zahlen von 1 bis 12 sind,
- p Null oder 1 ist,
- q Null oder eine ganze Zahl von 1 bis 12 bedeutet,
- Z -O- oder -NH- bedeutet,
- R¹ Wasserstoff oder eine C₁₋₄-Alkylgruppe bedeutet, und
- R² Wasserstoff oder eine Methylgruppe bedeutet.

2. Verbindungen nach Anspruch 1, die der Formel (1) oder (2) entsprechen, dadurch gekennzeichnet, daß die Gruppen R Alkylgruppen bedeuten.

3. Verbindungen nach Anspruch 2, dadurch gekennzeichnet, daß die Gruppen R Methyl, Ethyl, Propyl, n-Butyl, n-Octyl oder 2-Ethylhexyl bedeuten.

4. Verbindungen nach Anspruch 3, dadurch gekennzeichnet, daß die Gruppen R Methylgruppen sind.

5. Verbindungen nach einem der vorhergehenden Ansprüche, die der Formel (1) entsprechen, dadurch gekennzeichnet, daß die Gruppen B Alkyl bedeuten.

6. Verbindungen nach Anspruch 5, dadurch gekennzeichnet, daß die Gruppen B Methyl, Ethyl, Propyl, n-Butyl, n-Octyl oder 2-Ethylhexyl bedeuten.

7. Verbindungen nach Anspruch 6, dadurch gekennzeichnet, daß die Gruppen B Methylgruppen sind.

8. Verbindungen nach einem der vorhergehenden Ansprüche, die der Formel (1) entsprechen, dadurch gekennzeichnet, daß r im Bereich von 0 bis 3 und s im Bereich von 0 bis 3 liegt.

9. Verbindungen nach einem der Ansprüche 1 bis 4, die der Formel (2) entsprechen, dadurch gekennzeichnet, daß t + u im Bereich von 3 bis 5 liegt.

10. Verbindungen nach Anspruch 1, die der Formel (3) entsprechen, dadurch gekennzeichnet, daß die Gruppen R' Alkylgruppen sind, die unter Methyl, Ethyl, Propyl, n-Butyl, n-Octyl oder 2-Ethylhexyl ausgewählt sind.

11. Verbindungen nach Anspruch 10, dadurch gekennzeichnet, daß die Gruppen R' Methyl bedeuten.

12. Verbindungen nach einem der vorhergehenden Ansprüche, dadurch gekennzeichnet, daß n nicht Null und vorzugsweise 1 oder 2 ist und X unter Methyl, tert.-Butyl oder C₁₋₄-Alkoxy, insbesondere Methoxy, ausgewählt ist.

13. Verbindungen nach einem der vorhergehenden Ansprüche, dadurch gekennzeichnet, daß m nicht Null ist und Y -O- oder -NH- bedeutet.

14. Verbindungen nach einem der vorhergehenden Ansprüche, dadurch gekennzeichnet, daß p nicht Null ist und R¹ Wasserstoff bedeutet.

15. Verbindungen nach einem der vorhergehenden Ansprüche, dadurch gekennzeichnet, daß q im Bereich von Null bis 3 liegt.

16. Verbindungen einem der vorhergehenden Ansprüche, dadurch gekennzeichnet, daß die Bindung der Kette -(Y)ₘ-(CH₂-CHR¹)ₚ-(CH₂)_{q}-Z-CO-CHR²-CH₂- an die Benzotriazoleinheit in 3-, 4-, 4'- oder 5-Stellung erfolgt.

17. Verbindungen nach Anspruch 16, dadurch gekennzeichnet, daß die Bindung in 3-, 4-, oder 5-Stellung erfolgt.

18. Verbindungen nach einem der vorhergehenden Ansprüche, dadurch gekennzeichnet, daß die Bindung des Substituenten X an die Benzotriazoleinheit in 3-, 4-, 4'-, 5- und/oder 6-Stellung erfolgt.

19. Nichttherapeutische Verwendung der Verbindungen der Formeln (1) bis (3) als im UV-A-Bereich und UV-B-Bereich wirksame Sonnenschutzfilter.

20. Kosmetische Zusammensetzungen, insbesondere um UV-Strahlung zu filtern, dadurch gekennzeichnet, daß sie in einem kosmetisch akzeptablen Träger eine wirksame Menge mindestens einer Verbindung nach einem der Ansprüche 1 bis 18 enthalten.

21. Kosmetische Zusammensetzungen nach Anspruch 20, dadurch gekennzeichnet, daß der kosmetisch akzeptable Träger mindestens eine Fettphase oder ein organisches Lösungsmittel umfaßt.

22. Kosmetische Zusammensetzungen nach Anspruch 21, dadurch gekennzeichnet, daß der Träger in Form einer Öl-in-Wasser- oder Wasser-in-Öl-Emulsion und vorzugsweise in Form einer Öl-in-Wasser-Emulsion vorliegt.

23. Kosmetische Zusammensetzungen nach einem der Ansprüche 20 bis 22, dadurch gekennzeichnet, daß der Mengenanteil der Filterverbindung(en) im Bereich von 0,1 bis 20 Gew.-%, bezogen auf das Gesamtgewicht der Zusammensetzung, liegt.

24. Kosmetische Zusammensetzungen nach Anspruch 23, dadurch gekennzeichnet, daß der Mengenanteil im Bereich von 0,5 bis 10 Gew.-% liegt.

25. Nichttherapeutisches Verfahren zum Schutz der Haut und/oder der Haare gegen UV-Strahlung und insbesondere Sonnenlicht, dadurch gekennzeichnet, daß es darin besteht, auf die Haut und/oder die Haare eine wirksame Menge mindestens einer Verbindung oder Zusammensetzung nach einem der vorhergehenden Ansprüche aufzutragen.
